# EUROPEAN PATENT SPECIFICATION

(11) **EP 3 195 825 B1**
(45) Date of publication and mention of the grant of the patent: **31.07.2019**
(21) Application number: 15784473.9
(22) Date of filing: 17.09.2015
(51) Int. Cl.: A61C 8/00

(54) **DENTAL IMPLANT**
ZAHNIMPLANTAT
IMPLANT DENTAIRE

(30) Priority: 17.09.2014 PT 10789014
(43) Date of publication of application: 26.07.2017
(73) Proprietor: Universidade do Minho, 4704-3553 Braga (PT)
(72) Inventor: FERNANDES CARVALHO, Sandra Maria, 4710 - 057 Braga (PT); ALMEIDA ALVES, Cristiana Filipa, 4710 - 057 Braga (PT)
(74) Representative: Patentree
(86) International application number: PCT/IB2015/057171
(87) International publication number: WO 2016/042515

(56) References cited:
- WO-A1-2008/115883
- WO-A1-2013/119772
- WO-A2-2006/104644
- CN-U- 203 183 060

## Description

### Technical domain

The present disclosure is part of the biomedicine field and particularly of dental implants. More specifically, the present disclosure relates to a dental implant comprising a tantalum coating nanostructured by anodization and the method for its production.

The dental implant disclosed on the present disclosure allows a faster and effective osseointegration when compared with other commercial dental implants.

### Background

Currently, the commercial dental implants comprise essentially titanium (Ti) materials. However, it is generally known that the use of this material is not able to eliminate the occurrence of biological complications after their insertion into the human body.

The slow osseointegration of titanium dental implants leads to slow loading of the implant by the artificial crown, thus requiring a long non-operational time (around 6 months, depending on the patient). But the most relevant issue is related to the long non-operational time associated with the slow osseointegration, which enhances the probability of microorganisms entrance on the oral environment close to the surgical site, specifically to the surface of the dental implant. This adhesion and possible microbial colonization can induce the appearance of infections with consequent post-surgical complications. These infections are the main responsible for the short, medium and long term failure of the dental implants. The failure due to periimplantis disease is one of the most common diseases responsible to dental implant failure. Periimplantis disease is related with the late infection of the adjacent soft bone tissue that makes the bone recede. Consequently, a decrease in the mechanical anchorage to the implant is observed blocking the osseointegration. This decrease of the mechanical anchorage, when the soft-hard tissue recedes, leads to the "grey" Ti exposure with consequent poor aesthetics. Additionally, the existence of micro-mobility of dental implant, during the long non-functional time, induced by the inefficient healing and slow osseointegration, has a negative effect. The existence of micro-movements in long term is a problem which can result in loss of the implant. The resulting micro-mobility leads to a tensile and shear stresses on the surface of the implant that induce the encapsulation of the implant with fibrous tissue which also delays the osseointegration.

The osseointegration of dental implants is divided into two phases: the first phase is ensured by mechanical anchorage that derives from the design of the implant and the host bone structure. Over time, the primary mechanical connection decreases towards the biological anchorage, which is related to the interface formed between the implant surface and the bone.

The interaction between the bone and the implant surface is dynamic and results of the bone anchorage and stability of the biomaterial. The primary stability between the implant and the bone needed to achieve a successful implantation is characterized by a rigid fixation and absence of movement. This contact is influenced by several factors including the material, the design of the artificial structure and surface finish.

As a result, it is urgent to develop implants to improve the osseointegration, i.e., improve the structural, functional and direct connection between the living tissue, bone, and the surface of the dental implant that will subsequently be subjected to functional loads (masticatory load), to improve biological anchorage. To improve osseointegration, the materials to be used in the dental implants production have to be bioactive in order to enable the formation of a strong chemical bond with the bone.

As previously mentioned, nowadays dental implants are usually fabricated using titanium (Ti) based materials due to its biocompatibility and do not cause allergic reaction. These titanium based implants can be produced using commercially pure titanium or using a titanium alloy. The most common titanium alloy is titanium-6aluminium-4vanadium alloy (Ti6Al4V) due to its higher corrosion resistance. Commercially pure titanium (CP Ti) may be produced in dense or porous (micro scale) form, to alter its bulk properties. For instance, some studies show that the micrometer porosity can enhance primary fixation due to increase of mechanical anchorage by contact area increase, recompensing in some way the low bioactivity. The titanium reveal a slow osseointegration mostly due to the low capacity to form a strong chemical bond with living tissue, known as bioactivity, as well also shows a low stiffness which origin a low tribocorrosion resistance and subsequently in the presence of micro-mobility and an aggressive oral environment, could be critical.

These advantages or inconvenient, after the insertion of the implant, leads to the appearance of complications, already mentioned, that induce consequently high levels of dental implant failures and loss (according with M. Esposito et al., "Biological factors contributing to failures of osseointegrated oral implants (I). Success criteria and epidemiology," European Journal Oral Sciences, vol. 106, pp. 527-551, 1998*.*

About 47% before artificial crown implantation and 45% during the first year of use, due to the appearance of diseases or overload.

Another possibility to reduce the problems associated with the titanium could be the use of bioactive ceramic materials, such as synthetic hydroxyapatite (HA), bioglass or ceramic glass, since they are highly bioactive, for production of dental implants. Notice that, it is recognized that these materials reveal a faster healing with tolerance to the micro-mobility, during the first stages of the implantation, improving bone response to the implant. Still, the low tensile strength and fatigue resistance, as other mechanical properties, due to the fact that ceramic materials are fragile and brittle, makes impracticable the use of a fully ceramic implant.

One way to combine the good mechanical properties of metallic materials with good biological properties of bioactive ceramics could be the use of composite materials. However, it is extremely difficult to obtain a good interface between the bioactive material and the inert matrix, not allowing to have an effective charge transfer during mastication. Also the different behavior on the implant surface, due to the existence of two different materials with distinct properties, would be dramatic.

The deposition of a bioactive coating onto the metal surface is other possibility, as proposed by US 8545625 B2 patent application. Still, despite the different behavior at surface is not problematic, in this specific case it is crucial to have good adhesion between the metallic implant and the bioactive ceramic coating which is not always achieved since the deposited material has completely different properties when compared to the metallic base material.

The new generation of biomaterials pointed tantalum (Ta) as a promising material since, comparing to commercially pure titanium (Ti-cp), it is bioactive and presents high wettability and high surface energy which promotes the osseointegration. The substitution of commercial titanium dental implants by tantalum allows to promote the biological performance of implant in order to overtake the biological limitations which dental implants still show. The major drawback of tantalum is its high cost and high density, which makes bulk tantalum dental implants less cost effective than a dental implant coated with tantalum.

On the other hand, tantalum reveals a very high affinity to react with oxygen (O) which makes tantalum oxides its most common form. The Ta+O system may present different stoichiometries and tantalum pentoxide (Ta₂O₅) is the thermodynamic equilibrium tantalum oxide and therefore the most stable among several tantalum oxides. The higher surface energy of tantalum oxides stimulates the regeneration process in living tissues, and thus, increases the efficiency of the osseointegration, as well present high corrosion resistance.

According to Balla et al. in "Porous tantalum structures for bone implants: fabrication, mechanical and in vitro biological properties," Acta Biomaterialia, vol. 6, pp. 3349-3359, 2010*;* the cellular growth onto tantalum surfaces is more intense than onto titanium surfaces. The study compared the biological response of tantalum coatings, produced by LENSTM (Laser Engineered Net Shaping) and deposited onto titanium substrates, with titanium surfaces control. Cell viability tests were performed by measuring the enzymatic cellular activity (MTT assay) with hFOB (Fetal Osteoblast). The results of tantalum surface were significantly better regarding adhesion, cell growth and proliferation, when compared with titanium surfaces. In just 3 days, it was clear the higher bioactivity of tantalum, related with higher surface energy, when compared to the titanium, since the increase on the surface energy promotes the increase in cellular interactions which improve the biological fixation thought cell adhesion and proliferation.
To Y. X. Leng et al. in "The biocompatibility of the tantalum and tantalum oxide films synthesized by pulse metal arc vacuum deposition source," Nuclear Instruments and Methods in Physics Research Section B: Beam Interactions with Materials and Atoms, vol. 242, pp. 30-32, 2006*;* the use of tantalum and tantalum pentoxide (Ta₂O₅) in cardiovascular stents is the best alternative on the market. In fact, the superior bioactivity of these materials was evident when *in vitro* tests by culturing endothelial cells HUVEC (Human Umbilical Vein Endothelial Cells) were carried out and compared to stainless steel 316L behavior and commercially pure titanium with tantalum and pentoxide tantalum. Tantalum was deposited by plasma immersion ion implantation and tantalum pentoxide was obtained by annealing in an air atmosphere. After 24 hours of immersion, *in vitro* results shows on the tantalum and tantalum pentoxide surfaces an evident cell layer formation uniformly distributed. In contrast, the surface of stainless steel 316L and commercially pure titanium was hardly covered by cells. It was unequivocally the highest density of endothelial cells on the surface of tantalum and tantalum pentoxide when compared with the stainless steel 316L and the commercially pure titanium.

On the other hand, the surface modification aims to promote bone response favoring osseointegration. The chemistry and topography of the implant surface are determining factors in this process. The surface modification aims to improve primary stability by obtaining a higher contact area between the implant and the bone, accelerate osseointegration, growth and maturation of cells and reduce healing time, by controlling the interactions between the implant surface and bone cells, and finally, ensure successful fixation in areas with lower quality of host bone. This can be achieved by two main approaches: modification of the surface composition and / or modification of the surface topography.

Regarding chemical composition of surface, implants with oxidized surfaces play an important challenge in implantology considering that the biochemical interaction between the implant and the bone is in part determined by the chemical properties of the implant surface. The properties of the metal oxides are determined on the biochemical interactions since bone progenitor cells interact better with the oxide layer, forming a diffusion zone that promotes a stronger chemical bond. Besides changing the surface energy, the surface oxides also modify the kinetic adhesion, the type of adsorbed ions and consequently the space ions conformation. All of these factors affect the ability that the surface has to connect to neighboring cells.

The surface topography is related to the degree of roughness and the orientation of asperities. In fact, several authors claim that the roughness favors osseointegration, revealing a better biological response. It is recognized that roughness favors the initial stages of cell adhesion during the healing since several *in vivo* studies demonstrated that the increase in surface area increases the bone-implant contact improving the connection between them. Commercially, porous structures with dimensions in the micrometer order are accepted and associated to strong osseointegration due to the fact that improve the mechanical retention promoting a better fixation between the dental implant and the bone, as presented by EP 2143823 A2, EP 0560279 B1, EP 2554187 A1.

However, pores with nanometric dimensions are capable of biomimetic bone tissue since this is nanostructured besides promoting animal cells adhesion since they prefer surfaces with nano-roughness. The modification of implant surface topography at the nanoscale results in an increase of the interactions between the dental implant surface and the proteins, ions, biomolecules and bone cells, which will influence the adhesion, proliferation and differentiation of osteoblasts, accelerating osseointegration. Furthermore, the nanostructures present onto the implant surface will produce a bactericidal effect caused by mechanical deformation. Due to the nanostructured surface ability to attract and force the microbial cells to adapt to the topography, its subsequent deformation will consequently cause its death.

The modification of dental implants surface can be made with several surface treatments. These are divided into two major groups: subtraction or addition treatments. In case of subtraction there are numerous possible treatments such as sandblasting and etching, as disclosed by EP 2143823 A2 (but for applications in metal substrates based on titanium to orthopedic prostheses), electropolishing or anodization with the possibility of porosities formation.

Addition treatments consist in deposit the implant surface with typically bioactive materials, as exemplified by the deposition of bioactive ceramic materials which have characteristic biological response of living tissue. Within these materials, hydroxyapatite (HA) and calcium phosphate based compounds (Ca₃(PO₄)₂), which are the major constituents of natural bone, are the most used. The main complications identified in addition surface treatments are the risk of delamination of the added material. Due to the high difference between the properties of the metallic substrate (implant) and added ceramic (HA or Ca₃(PO₄)₂), they can crumble from each other. Consequently metallic implant movements occur as a result of rupture of the metallic implant - added ceramic materials interface and may result in the loss of the implant.

In the case of subtraction treatments, anodization is a chemical process which besides cheap is reproducible. They are characterized by voltage application between the anode (implant) and the cathode (lead, carbon, platinum, etc.) in the presence of an electrolyte, which generates the load transfer and ions causing consequently the growth of a surface oxide onto the implant surface. Typically this process is associated with an increase of the microscopic surface roughness and possible formation of nanometer-sized pores. The anodizing process may be performed in a single step or in more steps, according to the objectives. The anodizing process with two steps allows to have a high control over the morphology of the surface. In the first anodization, the formation of some uniform roughness occurs over all the surface. On the second anodization it is predicted to have a controlled pore growth which will result in an uniform surface roughness and controlled porosity well distributed. This specific anodization process approach has been the most widely used, particularly regarding biomedical applications, due to the uniformity and high control of the surface obtained and besides, it allows to have the topography surface modification (pore formation) and also chemical surface modification (formation of an oxide).

WO 2013119772 A1 patent application proposes the deposition of tantalum-based coatings on substrates whose surface contains titanium dioxide nanostructures. Notice that the nanostructures for dental implants in that document are based on titanium, which shows lower biological properties compared to tantalum. Furthermore, WO 2013119772 A1 proposes the production of nanostructures prior to deposition of the tantalum coating, unlike the present patent application which proposes a different layers configuration. Firstly, the tantalum based coating is deposited onto the dental implant and after the nanostructures are produced on the surface.

WO 2013119772 A1 application proposes the use of nanostructures of a different material from the coating (titanium dioxide nanostructures, TiO₂, and above it a tantalum coating). In the present disclosure, the nanostructures are totally based on tantalum which has an excellent osseointegration. In this case, contrary to WO 2013119772 A1, the morphology of nanostructures does not change with post-processing. Contrarily, WO2013119772 A1 document propose a coating deposition on top of the nanostructures that may mask / modify the morphology of the final surface.

The use of an entire functional tantalum based surface (tantalum based coating + tantalum oxide nanostructures), which is more bioactive itself, together with a nanostructured tantalum oxide improve the biological properties of implant.

The prior art technologies propose a coating deposition on top of the nanostructures masking its morphology, i.e., the post-coating deposition onto nanostructures negatively alter the morphology that was produced to improve bone cells adhesion. Furthermore, the modified surface for improving osseointegration is composed of two distinct materials. This difference of properties along with the surface mask may compromise the interaction with bone cell and thus, does not carry out a uniform and effective osseointegration.

These facts are described to illustrate the technical problems solved by the achievements of this document.

### General Description

The present disclosure combines two approaches in order to synergistically promote the osseointegration - by developing a bioactive and a nanostructured surface. The bioactive surface will be achieved through the application of tantalum, a material with improved biological properties in relation to the commercially used one, titanium. The nanostructured surface is obtained through the chemical and topographical modification of a tantalum based coating, through the growth of a nanoporous tantalum oxide, which allows to foster the biological response and biomimetic the osseous tissue in order to enhance the bonding between the implant surface and the bone. Simultaneously, the reduction of healing time reduces the probability of bacterial adhesion and colonization and consequently the occurrence of diseases.

The present disclosure is placed in the biomedicine field, more specifically in the field of surface modification of dental implants. The dental implants presently commercialized still show some biological limitations in short, medium and long range time, being the present focus of this disclosure the replacement by implants with bioactive and nanostructured surfaces based on tantalum.

In the present disclosure tantalum coatings are used, since this material shows good mechanical and biological properties, namely, excellent corrosion resistance as well as excellent bioactivity, which is not found in other metallic biomaterials, which allows to promote the osseointegration. In addition, with this disclosure a material with a nanostructured topography is shown, which enables to biomimetic the osseous tissue with consequent enhanced interaction between the surface and proteins, ions, biomolecules and cells, which will influence the adhesion, proliferation and differentiation of osteoblasts, thus accelerating the osseointegration. By tailoring the nano-roughness the differentiated osseous growth can be ruled.

More specifically, the present disclosure comprises a bioactive surface composed by a coating based on tantalum which is deposited over an implant, preferentially a metallic one, and through an anodization process which creates nanostructures in the tantalum coating which can comprise nanopores, nantubes or nanowires. The presence of nanostructures in the surface is an asset since for one hand they can set the osseous growth, and in other hand since the nanostructures are tantalum based they can enhance the osseointegration by increasing the contact area of this material with the body tissues/fluids. It should be pointed out that the nanostructures morphology is not changed after their processing.

In order to obtain a bioactive surface the tantalum based coating, which is deposited over the implant, comprises a chemical composition between 5-100 atomic percent of tantalum and 0-95 atomic percent of oxygen. The tantalum target used for the coating deposition has a purity degree which varies between 95-100 % of tantalum.

The present disclosure is related with a dental implant which comprises a tantalum coating nanostructured through an anodization process. The implant now described with a nanostructured tantalum coating obtainable by anodization process keeps fully the multifunctional surface able to accelerate and improve the osseointegration, reducing the microbial infections, which results from the exposure to a nanostructured tantalum oxide surface.

In other embodiments, the nanostructures of the nanostructured tantalum coating are: nanopores, nanotubes, nanofilaments, nanocapilars and combinations thereof.

In other embodiments, the nanostructures might be oriented in vertical, perpendicular in the direction of the dental implant width. The growth of nanopores, nanotubes, nanocapilars or nanowires might be totally or partially oriented in vertical.

In some embodiments, the tantalum nanostructures may cover the whole of dental implant, or the major part or a part of the implant, depending on the final goal for the application.

In some embodiments, the tantalum based coating might be deposited in the whole substrate, in a part of the substrate or in most of the substrate (implant surface) that constitutes the dental implant, depending on the final goal for the application.

In other embodiments, the nanostructured tantalum coating onto dental implant might comprise a thickness between 0.1-100 µm in order to modify its surface and aftermost produce the nanostructures and maintain a considerable thickness of a dense coating. With this goal it is aimed to guarantee the adhesion between de nanostructures and the tantalum coating and the adhesion between the coating and the implant, in order to not expose one material with different properties in relation to the bioactive and nanostructured coatings properties, the implant, to the biological medium.

In other embodiments, the nanopores of nanostructured tantalum coating onto dental implant might comprise a diameter in range between 1-3000 nm, depending of the envisaged final goal for the application. Preferentially and depending on the type of host cells, the adhesion of the osseous cells to the nanostructures might be maximized when the diameter ranges between 10-150 nm.

In other embodiments, the nanofilaments, nanocapilars or nanotubes of the nanostructured tantalum coating of the dental implant might comprise a height between 10-2000 nm, depending on the envisaged final goal for the application. Preferentially and depending on the type of host cells, the adhesion of the osseous cells to the nanostructures might be maximized when the height varies between 10-500 nm.

The nanostructures dimensions might be calculated by different methods such as conventional methods, namely electronic microscopy-scanning electron microscopy (SEM) or SEM micrography.

In other embodiments, the tantalum coating over the dental implant body might comprise between 5 to 100 atomic percent of tantalum.

In some embodiments, the material in the body of the dental implant is selected from a list consisting of: titanium or its alloys, tantalum or its alloys, steel alloy, cobalt-chromium-molybdenum alloy, polymeric materials, ceramic materials, composite materials, and combinations thereof, among others.

In some embodiments, the tantalum coating is selected from a list consisting of: tantalum, tantalum nitride, tantalum carbide, tantalum carbonitride, tantalum oxide, and mixtures thereof, among others. Although the coating is preferentially based in tantalum it might be replaced by pure tantalum, nitride/carbide/carbonitride of tantalum, or other alloy based on tantalum as long as the chosen material can be submitted to a surface treatment through anodization.

In other embodiments, the material of the implant body might be dense or porous.

In other embodiments, the coating deposition might be over a substrate of commercially pure titanium, titanium alloy, pure tantalum, tantalum alloy, stainless steel AISI 316L, cobalt-chromium-molybdenum alloy, polymer, ceramic, composite material or other biomaterial.

In some embodiments, dental implants might be produced in commercially pure titanium with a nanostructured tantalum layer.

In another embodiments, preferentially a pivot supported by the implant with a metallic substrate in commercially pure titanium, titanium alloy, stainless steel 316L, cobalt-chromium-molybdenum (Co-Cr-Mo) alloy or other biomaterial, is produced with a nanostructured tantalum layer.

Additionally in another preferential embodiment, a femoral rod, based in a hip prosthesis, with a metallic substrate in titanium alloy (Ti6Al4V) or an alloy of cobalt-chromium-molybdenum (Co-Cr-Mo), is produced with a nanostructured tantalum layer.

In another preferential embodiment, a biomedical application is produced with a substrate of commercially pure titanium, titanium alloy, pure tantalum, tantalum alloy, stainless steel 316L, cobalt-chromium-molybdenum (Co-Cr-Mo) alloy, polymer, ceramic, composite material or other biomaterial, with a nanostructured tantalum layer.

In one preferred embodiment, a biomedical application is produced with a nanostructured tantalum layer, which is composed by the tantalum coating with a nanostructured tantalum oxide, wherein nanostructured biological elements, osseous growth factors, medicines, or any other elements might be incorporated that might favor the osseointergration and/or prevent the microbial colonization and the appearance of infections.

The present disclosure also concerns a method for the deposition of a nanostructured tantalum coating over the body of the dental implant, comprising the following steps:
cleaning of the dental implant as previously mentioned;
deposition of a tantalum based coating over the dental implant body;
anodization of a tantalum based coating in order to obtain nanostructures, by applying a voltage difference below 150 V, wherein the anodization electrolyte comprises a mixture of H₂SO₄ and HF.

In other embodiments, the nanostructured tantalum coating might be realized with the use of a power source applied in continuous mode, alternating mode or in radiofrequency.

In other embodiments, the tantalum based coating might be deposited by physical vapor deposition, or chemical vapor deposition, or sol-gel or spin-coating.

In other embodiments, the current density applied to the power source used during the deposition is comprised in a range between 0.01-15 mA/cm².

In other embodiments, the deposition temperature might vary between 0-500 ºC, preferentially in a range between 180º-220 ºC.

In other embodiments, the negative bias voltage applied during the deposition might vary between 0 to -300 V, preferentially between -50-(-150 V).

In other embodiments, the deposition pressure might vary between 0.5 - 1.5 Pa.

In other embodiments, the anodization might comprise one single anodization step or it might comprise two anodization steps, this means, the nanostructures resulting from anodization process might be obtained using one single anodization step or they might be obtained with two anodization steps on the tantalum based coating.

In other embodiments, the method might be characterized by further comprising the following steps:
performing a chemical treatment with at least one of the following compounds NaOH, H₃PO₄, CrO₃ and combinations thereof;
submitting the surface to a second anodization, with a voltage difference inferior to 150 V and wherein the anodization electrolyte comprises a mixture of H₂SO₄ and HF, in order to standardize the surface and form pores;
performing a second chemical treatment to the surface, with an electrolyte that comprises at least one of the following compounds NaOH, H₃PO₄, CrO₃ and combinations thereof.

In other embodiments, the anodization temperature might be between 0-150 ºC.

In other embodiments, the time of each anodization might be up to 60 min, preferentially between 1-15 min, and more preferentially in a range between 2-10 min.

In other embodiments, the anodization electrolyte composition might comprise 5-98 % v/v of H₂SO₄ electrolyte.

In other embodiments, the anodization electrolyte composition comprises inferior to 60% v/v of HF electrolyte, preferentially between 2-10% v/v of electrolyte.

In other embodiments, the electrolyte composition for chemical treatment might contain up to 50 % v/v of NaOH solution.

In other embodiments, the electrolyte composition for the chemical treatment might contain up to 50 % v/v of H₃PO₄ solution.

In other embodiments, the electrolyte composition for chemical treatment might contain up to 50 % v/v of CrO₃ solution.

In other embodiments, the temperature of chemical treatments might be comprised between 0-150 ºC.

In other embodiments, the time of each chemical treatment might vary between 0.1-600 min, preferentially 180 min, more preferentially 2-30 min.

In summary, the tantalum composes the bioactive material which promotes the osseointegration. In combination with the tantalum oxide nanostructures, which mimetic the osseous tissue, the biological response of dental implant is promoted, reducing the infections appearance.

The coating deposition might be over a substrate of pure titanium, titanium alloy, pure tantalum, tantalum alloy, stainless steel 316L, cobalt-chromium-molybdenum (Co-Cr-Mo) alloy, polymer, ceramic, composite material or other biomaterial.

In terms of preferential embodiments, for a metallic substrate this must be cleaned before deposition for 10 min in consecutive ultrasonic baths on the following liquids: detergent + distilled water, acetone and ethanol. In each liquid the implant must be washed with distilled water and at the end dried in a hothouse at 60 ºC.

In other preferential embodiment, an acid etch must be performed taking into account the metallic substrate used.

In one preferred embodiment, the cleaning method uses for a metallic substrate that consists in commercially pure titanium (degree 2: ASTM F67), a mixture of 10 ml HF (using a solution with a concentration of 40 % volume as a starting point) with 100 ml of HNO₃ (using a solution with a concentration of 65 % in volume as a starting point).

The deposition might be performed through one of the following deposition processes:
Physical vapor deposition (PVD) (in all its variant modes);
Chemical vapor deposition (CVD) (in all its variant modes);
Sol-gel method;
Spin-coating.

The preferential deposition process is sputtering, wherein the atoms are pulverized from the tantalum target, through the propagation of a linear momentum promoted by the collision of energetic argon particles with the target, which will condense in the substrate surface. The coatings growth occurs through the condensation of atom by atom on the substrate surface. The attainment of a defect, porous, crack, among others, free coating is secured through the application of a negative polarization to the substrate during the deposition.

The deposition method is generally characterized by the deposition of a coating with the use of a power supply, which might be applied in continuous mode, alternate mode or radiofrequency mode.

Before performing the deposition, it is recommended to proceed to a previous treatment (etching) in order to remove the maximum amount of impurities from the tantalum target surface and substrates surface, such as avoid the coatings and substrate (implant) contamination, thus reducing the coatings adhesion problems.

The etching is performed preferentially with a DC power supply, which current density varies between 0.05-5 mA/cm² applied to the target and a pulsed DC power supply, which current varies between 250-5000 mA, with a duration of the preferential pulse between 1250-1850 ns and a frequency in the power supply between 150-300 kHz, in the substrate-holder.

The duration of etching might vary between 150-1800 s, being the inert gas used preferentially argon and the work pressure might vary between 0.1-1.5 Pa.

After this previous treatment, the deposition of an interlayer of pure tantalum could be performed in order to improve the adhesion between the substrate and the functional coating, in case it is necessary. The interlayer might be titanium, or other material compatible with the substrate used and the envisaged coating, such as tantalum based composites. The interlayer is preferentially deposited with a DC power supply applied to the target and a pulsed DC power supply in continuous mode in the substrate holder. The DC power supply applied to the target has a current density which varies between 5-15 mA/cm² and the pulsed DC power supply connected to the substrate working in continuous mode has a negative polarization (voltage difference) which varies between (-50)-(-125 V).

The interlayer deposition duration time might vary between 100-1800 s and the inert gas used is preferentially argon and the working pressure might vary between 0.05-1 Pa.

In what concerns the deposition process, this is performed preferentially with a DC power supply applied to the target and a pulsed DC power supply, working in continuous mode, in the substrate-holder. The DC power supply applied to the tantalum has a current density which varies between 0.5-15 mA/cm² and in the pulsed DC working in continuous mode the applied negative polarization might vary between 0 -(-300) V, preferentially between (-50)-(-125) V. The deposition temperature might vary between 0-500 ºC, preferentially between 180-220 ºC and the respective deposition time vary according to the envisaged thickness, preferentially between 1800-15000 s. The substrate-holder rotation speed is about 5-15 rpm. The system base pressure must be in the range between 5x10⁻³-1x10⁻⁶ Pa. The inert gas used is preferentially argon and the reactive gas might be oxygen. The working pressure might vary between 0.05-1 Pa, preferentially 0.1 and 0.8 Pa. With the adjustment of this parameters it is possible to optimize the mechanical and tribological properties of the coating deposited following the technique.

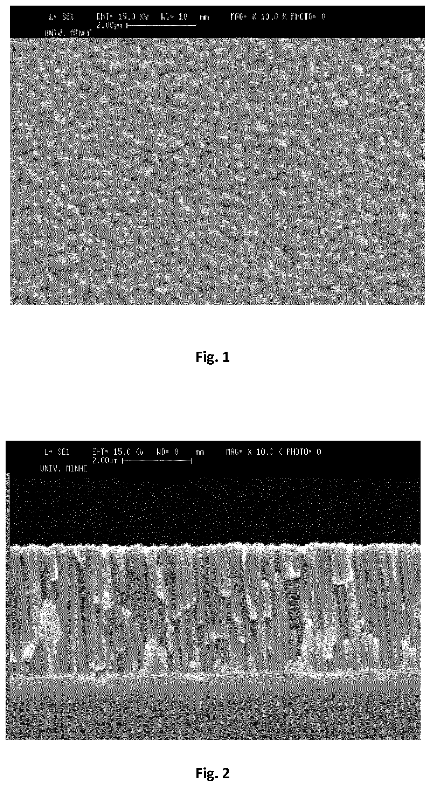

Once the coating is deposited over the implant, as schematized in Figures 1 and 2, it is important to evaluate the quality of coating adhesion to the substrate, through a peeling test.

Aftermost, the coated implant must be submitted to a new cleaning during 10 min in consecutive ultrasonic baths with the following liquids: distilled water, acetone and ethanol. Between the different liquids the implant must be cleaned with distilled water and in the end it might be dried in a hothouse at 60 ºC.

The implant surface is ready to be submitted to a topographical and chemical surface modification.

The nanostructures resulting from the anodization processes might be obtained through a single anodization step or might be obtained in two anodization steps performed in the tantalum coating.

The nanostructures are obtained preferentially by two anodization steps performed on the tantalum coating. The main goal of the anodization process is to guarantee the surface uniformization in order to control and promote the nanopores, nanotubes, nanocapilars or nanowires growth oriented partially or totally in the vertical.

The tantalum based nanostructures may cover one part, the major part or the totality of tantalum coating, depending on the final goal for the application.

The parameters that control the anodization steps are the temperature, time, potential, the electrolyte composition and concentration, and affect the anodization efficiency and also the shape/dimension of the nanostructure.

The electrolyte is one of the most important variables and its nature will influence the entire process. In fact, by knowing that the anodization is based in the surface oxidation, it is necessary that the electrolyte besides of being conductive, it contains ions with the ability to interact and promote the displacement of O²⁻ and OH⁻, responsible for the oxide formation. The electrolytes are in general strong acids, usually H₂SO₄, HF or H₃PO₄. Along with the choice of the electrolyte its concentration must be selected, which will determine the efficiency and velocity of the anodization process. The electrolyte choice will affect the porosity formation and it is specific for each material.

The temperature together with the electrolyte concentration is an important variable which allows to control the size of the pores being possible to increase the pore size with high temperatures (^{∼}85 ºC).

The anodization time and the applied voltage influence directly the thickness of the produced oxide. With the increase in the voltage the electrons number/flow in the material will increase, which will increase the anodization rate. Regardless, how much higher the applied voltage is higher the anodization rate is. With the increase in the anodization time, at a constant voltage, the oxide growth time is increased, which leads to an increase in the anodized thickness.

The anodization method is generally characterized by the surface modification of a material aiming the achievement of a oxidized material layer with the use of a power source, which might be applied in continuous mode, alternate mode, in pulsed mode or in radiofrequency mode.

In general terms, the anodization temperature for this process is comprised between 0-150 ºC and the anodization time might vary between 0.1-60 min. the voltage difference applied in the anodization might vary between 1-200 V and the electrolyte composition comprises between 5-98 % v/v of H₂SO₄ (from a solution with a concentration of 95-98 % volume) and 0-60 % v/v of HF (with a solution with a concentration ≥ 48 % volume).

At the beginning, between two anodization steps and/or in the final surface chemical treatments might be performed which allows to prepare the surface morphology to be anodized in order to handle the obtained topography. The chemical treatment electrolyte composition might comprise between 0-50 % in volume of NaOH (with a starting solution with a concentration ≥ 97% in weight), 0-50 % in volume of H₃PO₄ (from a starting solution with a concentration of ≥ 85% in volume) and/or 0-50 % in weight of CrO₃ (from a starting solution with a concentration ≥ 98% in weight). The chemical treatment temperature might vary between 0-150 ºC, wherein the time of chemical treatment might vary between 0.1-600 min.

With the adjustment of all above mentioned parameters it is possible to optimize the nanostructures obtained since they allow to tailor the size, shape and uniformity of the nanostructured topography obtained. The nanostructures obtained might be nanopores, nanotubes or nanowires.

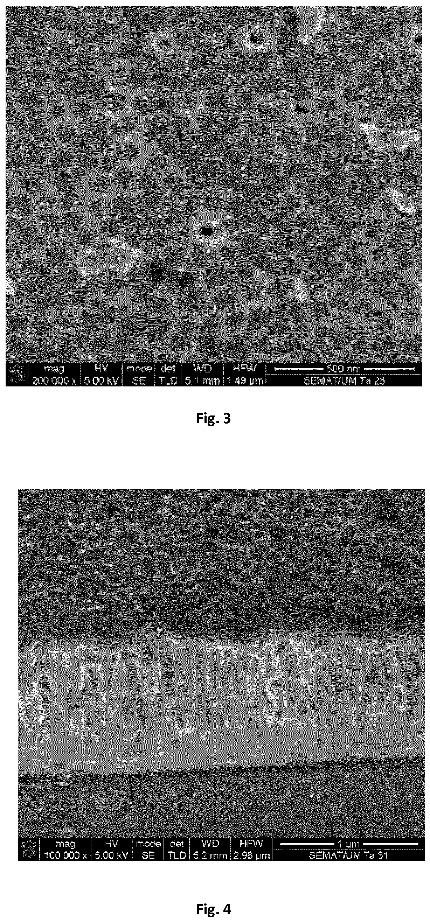

In the end a dental implant with a surface coated with a layer based on nanostructured tantalum, as represented in Figures 3 and 4, is obtained.

Optionally a pivot can be obtained, which is based on the implant, with a metallic titanium substrate with a nanostructured tantalum layer.

Along the disclosure and claims the word "comprise" and it variants do not intend to exclude other technical characteristics, such as other components or steps. Additional objects, advantages and characteristics of the disclosure will turn evident for expertizes in the technique after the examination of the description or might be learnt by the practice of the disclosure. The following examples and figures are provided as a way of illustrate and they do not intend to be limitative of the present disclosure. Besides, the present disclosure covers all the possible combinations of particular production forms or preferential ones here described.

### Brief description of the drawings

For an easier comprehension, the figures are joined in attachment, which represent preferential productions that do not aim to limit the object of the present disclosure.
Figure 1: SEM micrograph of the surface of a tantalum coating.
Figure 2: SEM cross-sectional micrograph of tantalum coating.
Figure 3: SEM micrograph of the nanostructures surface on tantalum oxide.
Figure 4: SEM micrograph of a cut section of the tantalum coating with tantalum oxide nanostructures with optimized parameters.
Figure 5: SEM micrograph of calcium phosphates adhered in the tantalum surface after 14 days of immersion in body simulated fluid at 37 ºC.

### Detailed Description

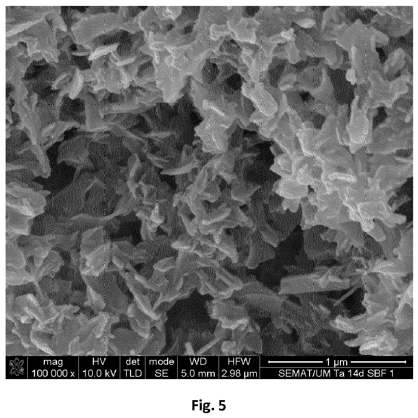

The present disclosure consists in the development of a bioactive nanostructured surface. The bioactive surface is obtained through the use of a material with good biological properties, the tantalum. The nanostructured surface is obtained through the chemical and topographical modification of the tantalum based coating with the growth of a nanostructured tantalum oxide which in ensemble allow to promote the biological response and biomimetic the osseous tissue, in order to strengthen the bonding between the implant surface and the bone, as presented in Table 1. In Table 1 the ratio of calcium and phosphorus (Ca/P) obtained on the surface developed in the present disclosure, tantalum surface, and on the commercially used material applied in dental implants, commercially pure titanium degree 2, after 2 and 14 days of immersion in body simulated fluid at 37 ºC are presented. This ratio allows to evaluate the evolution of Ca²⁺ and P⁵⁺ ions composition, which is related with the surface ability to exchange ions with the body fluid, being the Ca²⁺ and P⁵⁺ ions the main components of the bone. The higher this ability the better the biological performance of the material will be. After 14 days of immersion the Ca/P ratio of the developed surface is 1.57 a value very close to the Ca/P ratio in hydroxyapatite (the main mineral component of the bone), which according to the literature is around 1.63. In relation to commercially pure titanium degree 2 (ASTM: F67) it revealed a calcium phosphate adhesion significantly lower, which allow to conclude that the tantalum surface shows a significantly superior biological performance. The adhesion of calcium phosphates to the tantalum surface is presented in Figure 5.

Table: Evolution of Ca/P ratio after 14 days of immersion in body simulated fluid on tantalum surface developed in the present disclosure and on the commercially used titanium surface applied in dental implants.

| Ca/P ratio | Tantalum based surface | Commercially pure titanium degree 2 (ASTM: F67) |
|---|---|---|
| After 14 days of immersion | 1.57 | 0.95 |

The method uses, for a metallic substrate which consists in commercially pure titanium, a cleaning procedure before the deposition with 10 min in consecutive ultrasonic baths in the following liquids: detergent + distilled water, acetone and ethanol. Between each liquid the implant must be washed with distilled water and finally dried in a hothouse at 60 ºC.

For a metallic substrate consisting in commercially pure titanium (degree 2: ASTM F67), the method uses a mixture of 10 ml HF (with a starting solution with a concentration of 40 % volume) with 100 ml HNO₃ (with a starting solution with a concentration of 65 % in volume) in order to perform an acid stripping.

The tantalum coating deposition is performed by reactive magnetron sputtering.

For the coating deposition a tantalum target (99.99 % purity) is used. The working gas is argon.

The coating is deposited following a specific sequence of steps, which allows to maximize the quality of the coating produced. The depositions steps are:
Etching (cleaning of the target and substrates);
Coating deposition.

Yet, in the above mentioned steps some deposition parameters are kept constant, namely:
Temperature in the heating resistance in the deposition chamber - 473 K;
Substrate-holder rotation speed - 7 rpm;
Distance between target and substrate - 80 mm;
Pressure in the deposition chamber - ≈ 5x10⁻⁴ Pa;

Before starting the deposition it is fundamental to remove the maximum possible amount of impurities form the surface of the target, in order to not contaminate the coatings, and also the substrates, in order to reduce the adhesion problems in the coatings. The experimental conditions of this cleaning (etching) are presented in Table 2.

**Table 2: Etching experimental conditions.**

| | |
|---|---|
| Ar flow (sccm) | 80 |
| Target current density (mA/cm²) | 0.5 |
| Pulsed DC power supply current (A) | 0.48 |
| Pulse duration (ns) | 1536 |
| Pulsed DC power supply frequency (kHz) | 200 |
| Time (seconds) | 900 |
| Work pressure (Pa) | 0.6 |

Once the coating to be deposited is preferentially pure tantalum without addition of other chemical elements, there is no need to deposit any interlayer.

The deposition is performed immediately after the etching, in order to not have time enough for the dental implant surface and/or target surface get contaminated again. The experimental conditions of the coating deposition are presented in Table 3.

**Table 3: Coatings deposition experimental conditions.**

| | |
|---|---|
| Ar flow (sccm) | 60 |
| Target current density (mA/cm²) | 10 |
| Pulsed DC power supply potential (V) | -75 |
| Time (seconds) | 7200 |
| Work pressure (Pa) | 0.5 |
| O₂ flow (sccm) | 0 |

Once the coating is deposited on the implant, as presented in Figures 1 and 2 it is important to evaluate the quality of the coating adhesion, through peeling test.

Aftermost, the implant with the coating already deposited must be submitted to a new cleaning of 10 min in consecutive ultrasonic baths in the following liquids: distilled water, acetone and ethanol. Between each liquid the implant must be washed with distilled water and finally dried in a hothouse at 60 ºC.

The implant surface is now ready to be submitted to a topographical and chemical modification.

In one preferred embodiment, the nanostructures of tantalum oxide are obtained through the anodization technique in two steps.

More specifically, the anodization method comprises the following steps:
Perform a chemical treatment, preferentially at room temperature, with 0.3 M NaOH (using a starting solution with a concentration of ≥ 97% weight in NaOH), during 2 min, in order to remove the passivation oxide layer from the coating.
Unify the surface through a first anodization step with an electrolyte composed by a mixture of 1 M H₂SO₄ (using a starting solution with a concentration of 95-98 % volume) at room temperature (20 ºC) during 5 min. The applied voltage, preferentially, comprises 10 V.

Perform an intermediate chemical treatment to the surface with an electrolyte that comprises a mixture of 8 % (v/v) H₃PO₄ (using a starting solution with a concentration ≥ 85 % in volume) + 4 % (m/v) CrO₃ (using a starting solution with a concentration ≥ 98% in weight), at room temperature, during 10 min in order to create nucleation points in the future nanostructures.

Form the nanostructures through a second anodization step with an anodization electrolyte composed by a mixture of 16 M H₂SO₄ (using a starting solution with a concentration of 95-98 % volume) + 3 M HF (starting with a solution with a concentration of 4 8% in volume), at room temperature (20 ºC), during 2 min. The applied voltage, preferentially, comprises 15 V.

At the end a final chemical treatment is performed, with an electrolyte that comprises a mixture v/p of 8 % H₃PO₄ (starting with a solution with a concentration ≥ 85 % in volume) + 4 % CrO₃ (using a starting solution with a concentration ≥ 98% in weight) at room temperature (20 ºC), during 10 min in order to open the pore diameter.

At the end a dental implant is obtained with a surface coated with a nanostructured tantalum based layer, as presented in Figures 3 and 4.

The following claims define preferential productions.

## Claims

1. Dental implant comprising a nanostructure of tantalum and a nanostructured tantalum coating obtained by anodization.

2. Dental implant, according to the previous claim, wherein the nanostructures of the nanostructured tantalum coating are: nanopores, nanotubes, nanofilaments, nanocapilars and combinations thereof.

3. Dental implant, according to the previous claims, wherein the nanostructures are vertically oriented, perpendicularly in the implant's weight direction.

4. Dental implant, according to the previous claims, wherein the nanostructured tantalum coating recovers the totality of the implant.

5. Dental implant, according to the previous claims, wherein the nanostructured tantalum coating comprises a thickness between 0.1-100 µm.

6. Dental implant, according to the previous claims, wherein the nanostructured tantalum coatings nanopores comprise a diameter between 1-300 nm, preferentially 10-150 nm.

7. Dental implant, according to the previous claims, wherein the nanofilaments, nanocapilars or nanotubes in the nanostructured tantalum coating comprise a length between 10-2000 nm, preferentially 10-500 nm.

8. Dental implant, according to the previous claims, wherein the tantalum coating is selected from a list consisting of: tantalum, tantalum nitride, tantalum carbide, tantalum carbonitride, tantalum oxide, and mixtures thereof.

9. Dental implant, according to the previous claims, wherein the tantalum coating over the implant body comprises between 5 and 100 % of tantalum.

10. Dental implant, according to the previous claims, wherein the material in the implant body is selected from a list consisting of: titanium or its alloys, tantalum or its alloys, steel alloy, cobalt-chromium-molybdenum alloy, polymeric materials, ceramic materials, composite materials, and combinations thereof.

11. Dental implant, according to the previous claim, wherein the material of the implant body is dense or porous.

12. Method for the deposition of a nanostructured tantalum coating over the body of the dental implant, comprising the following steps:
cleaning of the dental implant;
deposition of a tantalum based coating on the implant body;
anodization of tantalum based coating in order to obtain nanostructures by applying a voltage difference inferior to 150 V, wherein the anodization electrolyte comprises a mixture of H₂SO₄ and HF.

13. Method, according to the previous claim, wherein the tantalum coating is selected from a list consisting of: tantalum, tantalum nitride, tantalum carbide, tantalum carbonitride, tantalum oxide and mixtures thereof.

14. Method, according to claim 13, wherein the anodization comprises two steps of anodization.

15. Method, according to the previous claim, **characterized by** further comprising the following steps:
performing a chemical treatment with at least one of the following compounds NaOH, H₃PO₄, CrO₃ and combinations thereof,
submitting the surface to a second anodization, with a voltage difference inferior to 150 V and wherein the anodization electrolyte comprises a mixture of H₂SO₄ and HF, in order to standardize the surface and form pores,
performing a second chemical treatment to the surface, with an electrolyte that comprises at least one of the following compounds NaOH, H₃PO₄, CrO₃ and combinations thereof.

## Patentansprüche

1. Zahnimplantat, umfassend eine Nanostruktur aus Tantal und eine nanostrukturierte Tantalbeschichtung, erhalten durch Anodisierung.

2. Zahnimplantat nach dem vorangehenden Anspruch, wobei die Nanostrukturen der nanostrukturierten Tantalbeschichtung sind: Nanoporen, Nanoröhren, Nanofilamente, Nanokapilare und Kombinationen davon.

3. Zahnimplantat nach den vorangehenden Ansprüchen, wobei die Nanostrukturen vertikal und senkrecht in Richtung des Gewichts des Implantats ausgerichtet sind.

4. Zahnimplantat nach den vorangehenden Ansprüchen, wobei die nanostrukturierte Tantalbeschichtung das gesamte Implantat bedeckt.

5. Zahnimplantat nach den vorangehenden Ansprüchen, wobei die nanostrukturierte Tantalbeschichtung eine Dicke zwischen 0,1-100 µm umfasst.

6. Zahnimplantat nach den vorangehenden Ansprüchen, wobei die nanostrukturierten Tantalbeschichtungen Nanoporen einen Durchmesser zwischen 1-300 nm, besonders bevorzugt 10-150 nm, umfassen.

7. Zahnimplantat nach den vorangehenden Ansprüchen, wobei die Nanofilamente, Nanokapilare oder Nanoröhren in der nanostrukturierten Tantalbeschichtung eine Länge zwischen 10-2000 nm, bevorzugt 10-500 nm, umfassen.

8. Zahnimplantat nach den vorangehenden Ansprüchen, wobei die Tantalbeschichtung ausgewählt ist aus einer Liste bestehend aus: Tantal, Tantalnitrid, Tantalcarbid, Tantalcarbonitrid, Tantaloxid, und Mischungen davon.

9. Zahnimplantat nach den vorangehenden Ansprüchen, wobei die Tantalbeschichtung auf dem Implantatkörper zwischen 5 und 100 % Tantal umfasst.

10. Zahnimplantat nach den vorangehenden Ansprüchen, wobei das Material in dem Implantatkörper ausgewählt wird aus einer Liste bestehend aus: Titan oder seinen Legierungen, Tantal oder seinen Legierungen, Stahllegierung, Kobalt-Chrom-MolybdänLegierung, polymeren Materialien, keramischen Materialien, Verbundwerkstoffen und Kombinationen davon.

11. Zahnimplantat nach dem vorhergehenden Anspruch, wobei das Material des Implantatkörpers dicht oder porös ist.

12. Verfahren zur Abscheidung einer nanostrukturierten Tantalbeschichtung auf dem Körper des Zahnimplantats, die folgenden Schritte umfassend:
Reinigung des Zahnimplantats;
Abscheidung einer Beschichtung auf Tantalbasis auf den Implantatkörper;
Anodisierung einer Beschichtung auf Tantalbasis, um Nanostrukturen durch Anlegen einer Spannungsdifferenz von weniger als 150 V zu erhalten, wobei der Anodisierungselektrolyt eine Mischung aus H₂SO₄ und HF umfasst.

13. Verfahren nach dem vorangehenden Anspruch, wobei die Tantalbeschichtung ausgewählt wird aus einer Liste bestehend aus: Tantal, Tantalnitrid, Tantalcarbid, Tantalcarbonitrid, Tantaloxid und Mischungen davon.

14. Verfahren nach Anspruch 13, wobei das Anodisieren zwei Anodisierungsschritte umfasst.

15. Verfahren nach dem vorangehenden Anspruch, **dadurch gekennzeichnet, dass** es ferner die folgenden Schritte umfasst:
Durchführen einer chemischen Behandlung mit mindestens einer der folgenden Verbindungen NaOH, H₃PO₄, CrO₃ und Kombinationen davon,
Behandlung der Oberfläche mit einer zweiten Anodisierung mit einer Spannungsdifferenz von weniger als 150 V und wobei der Anodisierungselektrolyt eine Mischung aus H₂SO₄ und HF umfasst, um die Oberfläche zu vereinheitlichen und Poren zu bilden,
Durchführen einer zweiten chemischen Behandlung der Oberfläche mit einem Elektrolyt, der mindestens eine der folgenden Verbindungen umfasst NaOH, H₃PO₄, CrO₃ und Kombinationen davon.

## Revendications

1. Implant dentaire comprenant une nanostructure de tantale et un revêtement de tantale nanostructuré obtenu par anodisation.

2. Implant dentaire, selon la revendication précédente, dans lequel les nanostructures du revêtement de tantale nanostructuré sont : des nanopores, nanotubes, nanofilaments, nanocapillaires et combinaisons de ceux-ci.

3. Implant dentaire, selon les revendications précédentes, dans lequel les nanostructures sont orientées verticalement, perpendiculairement dans la direction du poids de l'implant.

4. Implant dentaire, selon les revendications précédentes, dans lequel le revêtement de tantale nanostructuré recouvre la totalité de l'implant.

5. Implant dentaire, selon les revendications précédentes, dans lequel le revêtement de tantale nanostructuré comprend une épaisseur située entre 0.1-100 µm.

6. Implant dentaire, selon les revendications précédentes, dans lequel les nanopores des revêtements de tantale nanostructurés comprennent un diamètre situé entre 1-300 nm, de préférence 10-150 nm.

7. Implant dentaire, selon les revendications précédentes, dans lequel les nanofilaments, nanocapillaires ou nanotubes dans le revêtement de tantale nanostructuré comprennent une longueur située entre 10-2000 nm, de préférence 10-500 nm.

8. Implant dentaire, selon les revendications précédentes, dans lequel le revêtement de tantale est sélectionné à partir d'une liste qui consiste en : tantale, nitrure de tantale, carbure de tantale, carbonitrure de tantale, oxyde de tantale, et mélanges de ceux-ci.

9. Implant dentaire, selon les revendications précédentes, dans lequel le revêtement de tantale sur le corps de l'implant comprend entre 5 à 100% de tantale.

10. Implant dentaire, selon les revendications précédentes, dans lequel le matériau dans le corps de l'implant est sélectionné à partir d'une liste qui consiste en : titane ou ses alliages, tantale ou ses alliages, alliage d'acier, alliage de cobalt-chrome-molybdène, matériaux polymères, matériaux céramiques, matériaux composites, et combinaisons de ceux-ci.

11. Implant dentaire, selon la revendication précédente, dans lequel le matériau du corps de l'implant est dense ou poreux.

12. Méthode pour le dépôt d'un revêtement de tantale nanostructuré sur le corps de l'implant dentaire, comprenant les étapes suivantes :
nettoyage de l'implant dentaire ;
dépôt d'un revêtement à base de tantale sur le corps de l'implant ;
anodisation du revêtement à base de tantale afin d'obtenir des nanostructures en appliquant une différence de tension inférieure à 150 V, dans laquelle l'électrolyte d'anodisation comprend un mélange de H₂SO₄ et de HF.

13. Méthode, selon la revendication précédente, dans laquelle le revêtement de tantale est sélectionné à partir d'une liste qui consiste en : tantale, nitrure de tantale, carbure de tantale, carbonitrure de tantale, oxyde de tantale, et mélanges de ceux-ci.

14. Méthode, selon la revendication 13, dans laquelle l'anodisation comprend deux étapes d'anodisation.

15. Méthode, selon la revendication précédente, **caractérisée en ce qu'**elle comprend également les étapes suivantes :
réaliser un traitement chimique avec au moins un des composés suivants NaOH, H₃PO₄, CrO₃ et combinaisons de ceux-ci,
soumettre la surface à une seconde anodisation, avec une différence de tension inférieure à 150 V et dans laquelle l'électrolyte d'anodisation comprend un mélange de H₂SO₄ et de HF, afin de standardiser la surface et de former des pores,
réaliser un second traitement chimique à la surface, avec un électrolyte qui comprend au moins un des composés suivants NaOH, H₃PO₄, CrO₃ et combinaisons de ceux-ci.
